**Europäisches Patentamt**

**European Patent Office**

**Office européen des brevets**

(19)

(11) Veröffentlichungsnummer: **0 215 378**

**B1**

(12) **EUROPÄISCHE PATENTSCHRIFT**

(45) Veröffentlichungstag der Patentschrift:
**23.11.88**

(51) Int. Cl.⁴: **C 07 C 31/20,** C 07 C 29/15

(21) Anmeldenummer: **86112212.5**

(22) Anmeldetag: **04.09.86**

(54) **Verfahren zur Herstellung von Ethylenglycol.**

(30) Priorität: **14.09.85 DE 3532877**

(43) Veröffentlichungstag der Anmeldung:
**25.03.87 Patentblatt 87/13**

(45) Bekanntmachung des Hinweises auf die Patenterteilung:
**23.11.88 Patentblatt 88/47**

(84) Benannte Vertragsstaaten:
**BE DE FR GB IT NL**

(56) Entgegenhaltungen:
**EP-A-0 033 212**
**EP-A-0 055 668**
**EP-A-0 085 191**
**DE-A-2 746 245**
**DE-A-3 427 138**
**US-A-3 974 259**
**US-A-4 144 401**
**US-A-4 225 530**

(73) Patentinhaber: **BASF Aktiengesellschaft, Carl-Bosch- Strasse 38, D-6700 Ludwigshafen (DE)**

(72) Erfinder: **Maerkl, Robert, Dr., Schulstrasse 17, D-6701 Fussgoenheim (DE)**
Erfinder: **Bertleff, Werner, Dr., Neuzenlache 3, D-6806 Viernheim (DE)**
Erfinder: **Harder, Wolfgang, Dr., Bergwaldstrasse 16, D-6940 Weinheim (DE)**
Erfinder: **Kummer, Rudolf, Dr., Kreuzstrasse 6, D-6710 Frankenthal (DE)**

**Beschreibung**

Die vorliegende Erfindung betrifft ein verbessertes Verfahren zur Herstellung von Ethylenglycol aus Kohlenmonoxid und Wasserstoff in homogener flüssiger Phase unter erhöhtem Druck und bei erhöhter Temperatur in Gegenwart Rhodium enthaltender Katalysatoren sowie in Gegenwart von organischen Lösungsmitteln.

Diese sogenannte Direktsynthese von niederen Alkoholen aus CO und $H_2$, bei der neben dem hauptsächlich erwünschten Ethylenglycol u.a. Methanol und Ethanol gebildet werden, ist, sieht man von der erfindungsgemäßen Verbesserung ab, aus zahlreichen Veröffentlichungen allgemein bekannt, wobei als Lösungsmittel u.a. Alkanole wie Methanol, Ethanol, Propanol, Isobutanol und 2-Ethylhexanol genannt werden (s. z. B. US-A-3 974 259).

In jüngeren Patentveröffentlichungen wie der EP-A-0 085 191 werden Alkanole als Lösungsmittel dagegen nicht erwähnt; vielmehr wird dort ausdrücklich empfohlen, den Anteil des Ethylenglycols im Reaktionsgemisch unter 20 Gew.% und die Gesamtkonzentration von Ethylenglycol, Methanol und Ethanol unter 50 Gew.-% zu halten.

Nach der Lehre der EP-A-55 668 nimmt man die Direktsynthese des Gemisches aus Ethylenglycol, Methanol und Ethanol mit Hilfe von Rutheniumkatalysatoren vor, wobei empfohlen wird, dem Reaktionsgemisch wirksame Mengen derjenigen Alkohole zuzusetzen, deren Neubildung man zurückdrangen möchte. Wie die dortigen Beispiele indes zeigen, wirkt sich der Zusatz von Methanol oder Ethanol praktisch nicht auf die Ausbeute des Ethylenglycols aus.

Entsprechend der Aufgabe, die eingangs definierte Direktsynthese zur Herstellung von hauptsächlich Ethylenglycol zu verbessern, wurde indes wider Erwarten gefunden, daß man erheblich höhere Ausbeuten an Ethylenglycol erzielt, wenn im Reaktionsgemisch stets mindestens 50 Gew.-%, bezogen auf die Gesamtmenge des Gemisches, eines $C_2$-$C_{20}$-n-Alkanols vorliegen.

Aus wirtschaftlichen Gründen werden die $C_2$-$C_8$-Alkanole, also Ethanol, n-Propanol, n-Butanol, n-Pentanol, n-Hexanol, n-Heptanol und n-Octanol sowie auch deren Gemische bevorzugt, und unter diesen aus verfahrenstechnischen Gründen die in Wasser wenig löslichen $C_5$-$C_8$-Alkanole, da die geringe Wasserlöslichkeit es gestattet, die gebildeten Syntheseprodukte Ethylenglycol, Methanol und Ethanal durch eine einfache Extraktion mit Wasser aus dem Reaktionsgemisch zu gewinnen.

Da die erfindungsgemäß zu verwendenden Alkanole die Direktsynthese des Ethylenglycols begünstigen, sind möglichst hohe Alkanol-Konzentrationen im Reaktionsgemisch prinzipiell von Vorteil. Da dies aber zu unwirtschaftlich großen Volumina führen wurde, empfiehlt sich eine Anfangskonzentration von etwa 80 - 98 Gew.-%. Man laßt die Reaktion dann zweckmäßigerweise soweit fortschreiten, bis die Endkonzentration infolge des neu gebildeten Ethylenglycols 50 Gew.-%, vorzugsweise etwa 60 - 80 Gew.-%, beträgt. Weitere Lösungsmittel wie diejenigen, die in der EP-A-0 085 191 für die Zwecke der Direktsynthese aufgeführt sind, können mitverwendet werden, z. B. um die Löslichkeit des Katalysators zu erhöhen. Einige dieser Lösungsmittel wie offenkettige und cyclische Tetraalkylharnstoffe, Lactone sowie N-Aryl- und N-Alkylpyrrolidone und -imidazolidone, besonders N-Methylpyrrolid-2-on, 1,5-Dimethylpyrrolid-2-on und 1,3-Dimethylimidazolid-2-on haben einen begünstigenden Effekt auf die Reaktion und werden deshalb bevorzugt.

Als Katalysatoren verwendet man entweder Rhodium allein oder zusammen mit anderen Carbonylbildenden Metallen wie Ruthenium, Palladium, Platin, Iridium, Eisen, Nickel und besonders Cobalt, wobei der Anteil des Rhodiums mindestes 5 mol-% betragen sollte.

Da sich die aktiven Carbonylkomplexe während der Reaktion von selber bilden, kann man die Katalysatoren in metallischer Form oder vorzugsweise in Form ihrer Salze oder Komplexverbindungen wie den Carbonylkomplexen oder Komplexen mit beispielsweise Acetylaceton einsetzen.

Besonders bevorzugt werden gemäß der älteren Anmeldung P 34 27 138.4 Mischkatalysatoren oder Katalysatorgemische mit Rhodium und Cobalt als aktiven Metallen, in denen das Molverhältnis von Rhodium zu Cobalt etwa 20 : 1 bis 60 : 1 beträgt.

Die Rhodiumkonzentration bzw, die Gesamtkonzentration der carbonylbildenden Metalle im Reaktionsmedium kann im Prinzip beliebig sein, da sie im wesentlichen nur einen Einfluß auf die Reaktionsgeschwindigkeit und damit auf die Raum-Zeit-Ausbeute hat. Befriedigende Raum-Zeit-Ausbeuten werden in der Regel im Konzentrationsbereich von 0,01 - 0,5 Gew.-% Metall erzielt; höhere Konzentrationen bringen keine nennenswerten wirtschaftlichen Vorteile mehr, und bei geringeren Konzentrationen - etwa bis herab zu 0,005 Gew.-% verlangsamt sich die Reaktion entsprechend.

Wie es für die Direktsynthese allgemein bekannt ist, empfiehlt sich auch im vorliegenden Falle die Mitverwendung eines offenkettigen oder cyclischen tertiären Amins wie N-Methylmorpholin, da derartige Verbindungen als Liganden in die Katalysatoren eintreten und deren Stabilität bekanntermaßen erhöhen. Die Menge dieser Verbindungen beträgt vorzugsweise etwa 1 - 50 mol pro Mol der Zentralatome.

Für den Gesamtdruck empfiehlt sich ein Bereich von 300 - 3000, vorzugsweise 600 - 2000 bar, wobei der Partialdruck des Kohlenmonoxids vorzugsweise zwischen 20 und 80 %, besonders zwischen 30 und 60 %, des Gesamtdruckes liegt.

Gute Ergebnisse erzielt man bei Temperaturen von 180 - 280°C, wobei der Bereich von 200 - 240°C im allgemeinen zu bevorzugen ist.

Man kann die Reaktion nach den hierfür üblichen Techniken diskontinuierlich oder kontinuierlich vornehmen. Die Aufarbeitung der Reaktionsgemische erfolgt entweder destillativ oder, falls ein wasserunlösliches Alkanol

2

0 215 378

als Reaktionsmedium dient, besonders zweckmäßig durch Extraktion mit Wasser und anschließende Fraktionierung der Extraktphase.

Die letztgenannte Arbeitsweise bietet den Vorteil, daß man die zurückbleibende katalysatorhaltige Raffinatphase unmittelbar wieder für einen weiteren Reaktionsansatz verwenden kann, da das in die Raffinatphase gelangende Wasser bei der Reaktion nicht stört. Diese Arbeitsweise eignet sich verfahrenstechnisch besonders gut für den kontinuierlichen Betrieb.

Bei der rein destillativen Aufarbeitung, die im Falle der Verwendung wasserlöslicher Alkanole erforderlich ist, erhält man eine katalysatorhaltige Sumpfphase, die ebenfalls wieder in die Reaktion zurückgeführt werden kann.

Nach den bisherigen Beobachtungen bedingt die erfindungsgemäße Verwendung der Alkanole als Reaktionsmedium stets eine deutlich bessere Ausbeute an Ethylenglycol, verglichen mit der Arbeitsweise unter Verwendung geringerer Alkanolkonzentrationen oder unter Verwendung anderer Lösungsmittel bei jeweils gleichen sonstigen Reaktionsbedingungen, d.h. auf die sonstigen Reaktionsbedingungen kommt es im Hinblick auf die relative Verbesserung nicht an.

**Beispiel 1**

Es wurden jeweils 150 g einer Lösung aus

| | |
|---|---|
| 1,0 | g $Rh(CO)_2 \cdot$ Acetylaceton (= 0,4 g Rh) |
| 0,026 | g $Co_2(CO)_8$ (= 0,009 g Co) |
| 1,88 | g N-Methylmorpholin |
| a | g 1,5-Dimethylpyrrolid-2-on und |
| b | g Hexan-1-ol |

wobei a + b rd. 200 g beträgt, 5 Stunden lang bei 230°C und einem Gesamtdruck von 1 500 bar mit einem äquimolaren $CO/H_2$-Gemisch zur Reaktion gebracht.

Einzelheiten und Ergebnisse dieser Versuche sind der nachstehenden Tabelle zu entnehmen. Die Ausbeuten wurden hierbei gaschromatographisch ermittelt.

| Vers. Nr. | a [g] | b [g] | MeOH | EtOH | HO-$CH_2$-$CH_2$-OH |
|---|---|---|---|---|---|
| zum Vergleich | | | | Ausbeute [g] an | |
| 1 | 200 | 0 | 15,8 | 2,2 | 37,2 |
| 2 | 140 | 60 | 13,9 | 2,4 | 39,6 |
| 3 | 110 | 90 | 6,4 | 14,5 | 35,2 |
| erfindungsgemäß | | | | | |
| 4 | 60 | 140 | 15,0 | 11,0 | 60,4 |
| 5 | 20 | 180 | 14,5 | 9,9 | 54,1 |

Wie man erkennt, ist die Ausbeute an Ethylenglycol im Falle der Versuche 4 und 5 deutlich höher als im Falle der Vergleichsversuche.

**Patentansprüche**

1. Verfahren zur Herstellung von Ethylenglycol aus Kohlenmonoxid und Wasserstoff in homogener flüssiger Phase unter erhöhtem Druck und bei erhöhter Temperatur in Gegenwart Rhodium enthaltender Katalysatoren sowie in Gegenwart von organischen Lösungsmitteln, dadurch gekennzeichnet, daß man als organisches Lösungsmittel ein $C_2$-$C_{20}$-n-Alkanol verwendet und daß man die Konzentration dieses Alkanols, bezogen auf die Gesamtmenge des Gemisches, stets bei mindestens 50 Gew,-% hält.

2. Verfahren nach Anspruch 1, dadurch gekennzeichnet, daß die Alkanol-Konzentration zu Beginn der Reaktion 80 - 95 Gew.-% beträgt.

3. Verfahren nach den Ansprüchen 1 und 2, dadurch gekennzeichnet, daß man als Alkanol ein $C_2$-$C_8$-Alkanol verwendet.

4. Verfahren nach den Ansprüchen 1 bis 3, dadurch gekennzeichnet, daß man als Alkanol ein $C_5$-$C_8$-Alkanol verwendet.

5. Verfahren nach den Ansprüchen 1 bis 4, dadurch gekennzeichnet, daß man als Katalysator einen Rhodium/Cobalt-Mischkomplex oder eine Mischung aus einem Rhodium- und einem Cobaltkomplex verwendet, wobei das Molverhältnis von Rhodium zu Cobalt etwa 20 : 1 bis 60 : 1 beträgt.

3

**0 215 378**

**Claims**

1. A process for preparing ethylene glycol from carbon monoxide and hydrogen in homogeneous liquid phase under superatmospheric pressure and at elevated temperature in the presence of a rhodium-containing catalyst and in the presence of an organic solvent, wherein the organic solvent is a $C_2-C_{20}$-n-alkanol and the concentration of this alkanol, based on the total amount of the mixture, is constantly maintained at not less than 50 % by weight.

2. A process as claimed in claim 1, wherein the alkanol concentration at the start of the reaction is 80 - 95 % by weight.

3. A process as claimed in claims 1 and 2, wherein the alkanol is a $C_2-C_8$-alkanol.

4. A process as claimed in any of claims 1 to 3, wherein the alkanol is a $C_5-C_8$-alkanol.

5. A process as claimed in any of claims 1 to 4, wherein the catalyst is a rhodium/cobalt co-complex or a mixture of a rhodium and a cobalt complex, the molar ratio of rhodium to cobalt ranging from about 20 : 1 to 60: 1.

**Revendications**

1. Procédé de préparation d'éthylèneglycol à partir d'oxyde de carbone et d'hydrogène en phase liquide homogène sous pression élevée et à température élevée, en présence de catalyseurs contenant du rhodium et en présence de solvants organiques, caractérisé en ce qu'an utilise, comme solvant organique, un n-alcanol en $C_2-C_{20}$ et en ce qu'an maintient constamment la concentration de cet alcanol, par rapport à la quantité totale du mélange, à 50 % en poids au moins.

2. Procédé selon la revendication 1, caractérisé en ce que la concentration d'alcanol au début de la réaction s'élève à 80 - 85 % en poids.

3. Procédé selon la revendication 1 ou 2, caractérisé en ce qu'on utilise, en tant qu'alcanol, un alcanol en $C_2-C_8$.

4. Procédé selon l'une quelconque des revendications 1 à 3, caractérisé en ce qu'on utilise, en tant qu'alcanol, un alcanol en $C_5-C_8$.

5. Procédé selon l'une quelconque des revendications 1 à 4, caractérisé en ce qu'on utilise, comme catalyseur, un complexe mixte de rhodium/cobalt ou un mélange d'un complexe de rhodium et d'un complexe de cobalt, le rapport molaire du rhodium au cobalt étant compris entre 20 : 1 et 60 : 1 environ.

4